# EUROPEAN PATENT SPECIFICATION

(11) **EP 3 531 130 B1**
(45) Date of publication and mention of the grant of the patent: **13.04.2022**
(21) Application number: 17861349.3
(22) Date of filing: 17.10.2017
(51) Int. Cl.: G01N 33/543, G01N 33/545

(54) **IMMUNOASSAY METHOD AND ASSAY REAGENT**
IMMUNOTESTVERFAHREN UND TESTREAGENS
PROCÉDÉ DE DOSAGE IMMUNOLOGIQUE ET RÉACTIF DE DOSAGE

(30) Priority: 19.10.2016 JP 2016205056
(43) Date of publication of application: 28.08.2019
(73) Proprietor: Eiken Kagaku Kabushiki Kaisha, Taito-ku Tokyo 110-8408 (JP)
(72) Inventor: MURAKAMI Yusuke, Shimotsuga-gun Tochigi 329-0114 (JP)
(74) Representative: Altmann Stößel Dick Patentanwälte PartG mbB
(86) International application number: PCT/JP2017/037517
(87) International publication number: WO 2018/074467

(56) References cited:
- EP-A2- 0 202 081
- JP-A- H01 253 654
- JP-A- S55 116 259
- JP-A- S61 264 262
- JP-A- 2001 074 739
- JP-A- 2001 255 325
- JP-A- 2001 289 850
- JP-A- 2002 303 630
- JP-A- 2009 174 871
- US-A- 5 208 166
- US-A1- 2008 182 341
- KALINA RANGUELOVA ET AL: "Protein Radical Formation Resulting from Eosinophil Peroxidase-catalyzed Oxidation of Sulfite", JOURNAL OF BIOLOGICAL CHEMISTRY, vol. 285, no. 31, 25 May 2010 (2010-05-25) , pages 24195-24205, XP055695843, US ISSN: 0021-9258, DOI: 10.1074/jbc.M109.069054
- Bartels Else Marie ET AL: "Rheumatoid Factor and Its Interference with Cytokine Measurements: Problems and Solutions", Arthritis, vol. 2011, 22 June 2011 (2011-06-22), pages 1-7, XP55783123, ISSN: 2090-1984, DOI: 10.1155/2011/741071 Retrieved from the Internet: URL:http://downloads.hindawi.com/archive/2 011/741071.xml>

## Description

### Technical Field

The present invention is defined by the claims.

### Background Art

Since an immunoassay method of utilizing an antigen-antibody reaction between an antigen and an antibody can simply and quickly measure an immunologically active substance in a living body, such as a protein, it has been widely used in the diagnosis of various types of diseases in recent years. As such immunoassay methods, radioimmunoassay (RIA), enzyme immunoassay (EIA), fluorescence immunoassay (FIA), turbidimetric immunoassay (TIA), latex agglutination immunoassay (LIA), immunochromatography, etc. have been put to practical use.

However, there is a case where a specimen as a measurement target of the immunoassay method, contains an interference substance that interferes an antigen-antibody reaction. If such an interference substance is involved in an antigen-antibody reaction, it affects the measurement, and thus, a precise measurement cannot be performed. In particular, in the field of clinical tests, a precise diagnosis cannot be made. Accordingly, a method of reducing or suppressing the influence of an interference substance has been proposed so far.

For example, in order to solve the problem regarding interference effects due to the mixing of a rheumatoid factor, there has been proposed an immunoassay method of previously pre-treating a specimen with an animal-derived antibody binding to the binding site of a human rheumatoid factor, so as to decrease interference effects caused by the rheumatoid factor (Patent Literature 1), or an immunoassay method of keeping the pH of a reaction solution at pH 4.0 to 6.0, so as to suppress interference effects caused by a rheumatoid factor (Patent Literature 2), or the like.

Moreover, in order to decrease or suppress interference effects caused by an interference substance, a method of using F(ab')₂, in which an Fc portion of an antibody used in an immunological measurement has been removed by an enzyme reaction, has been proposed (Patent Literature 3). However, this immunoassay method has been problem in that the method can be used only in the case of measuring an antigen, and further, in that complicated steps, such as an enzyme reaction and purification, are necessary for production of F(ab')₂, and they cause an increase in costs or a difference among production lots.

Furthermore, a method of suppressing interference effects caused by a heterophile antibody by utilizing polymerized or agglutinated antibody molecules has been proposed (Patent Literature 4). However, in order to completely suppress interference effects caused by a heterophile antibody, large amounts of polymerized or agglutinated antibody molecules are necessary, and they cause a problem regarding high costs.

According to the methods of suppressing interference effects caused by interference substances, which are described in the aforementioned publications, the influence of interference substances has been certainly improved. However, when a high concentration of interference substance has been contained in a specimen to be measured, the effect of suppressing the interference effects caused by the interference substance has been insufficient.

A method of using a sulfite, which can suppress interference effects caused by an interference substance even in a case where the interference substance is contained in a high concentration in a specimen to be measured, has been proposed (Patent Literature 5). Sulfite is advantageous in that it is extremely inexpensive, in that it does not lead to an increase in costs even if it is used in a concentration that is sufficient for suppressing the influence of a high concentration of interference substance, and also in that it hardly affects an antigen-antibody reaction.

Nevertheless, since the sulfite is gradually oxidized to a sulfate by dissolved oxygen contained in a measurement reagent or oxygen in the air, the effect of suppressing interference effects caused by an interference substance is decreased over time. That is to say, the sulfite has problem that, when the sulfite is stored in an opened state in a reagent storage or the like for its use in an automatic analyzer, the concentration of the sulfite is decreased over time, and as a result, it cannot sufficiently suppress the influence of an interference substance. US5208166 describes reactive chitosan coated articles and a test kit for an immunoassay. EP0202081 describes a single step heterogeneous assay and a kit. Ranguelova et al. (Journal of Biological Chemistry 2010, vol. 285(31), p. 24195-24205) describe protein radical formation resulting from eosinophil peroxidase-catalyzed oxidation of sulfite. Bartels et al. (Arthritis. 2011;2011:741071. doi: 10.1155/2011/741071. Epub 2011 Jun 22.) describe rheumatoid factor and its interference with cytokine measurements.

### Citation List

### Patent Literature

Patent Literature 1: JP Patent Publication (Kokai) No. 7-012818 A (1995)
Patent Literature 2: JP Patent Publication (Kokai) No. 8-146000 A (1996)
Patent Literature 3: JP Patent Publication (Kokai) No. 54-119292 A (1979)
Patent Literature 4: JP Patent Publication (Kokai) No. 4-221762 A (1992)
Patent Literature 5: JP Patent Publication (Kokai) No. 2001-074739 A

### Summary of Invention

### Technical Problem

The present inventor has found that, by setting the pH of a measuring reagent comprising a sulfite at pH 7.0 or less, the stability of the sulfite can be improved under an air atmosphere, the effect of suppressing interference effects caused by an interference substance can be maintained for a long period of time, and further, the interference effects of an extremely high concentration of heterophile antibody can be sufficiently suppressed.

However, the present inventor has faced such a problem that, by setting the pH of a measuring regent comprising a sulfite at pH 7.0 or less, when a hemolytic specimen is measured, it is influenced by hemoglobin, and as a result, a precise measurement cannot be carried out with such a hemolytic specimen.

Such influence of hemoglobin causes a serious problem, particularly, to immunoassay methods that do not involve B/F separation (Bound/Free separation), such as turbidimetric immunoassay (TIA) or latex agglutination immunoassay (LIA), which are based on an immune agglutination reaction method.

Accordingly, the present invention has been made by focusing the aforementioned circumstances, and it is an object of the present invention to provide an immunoassay method, which can sufficiently suppress interference effects caused by an interference substance for a long period of time, even in a case where a measurement specimen contains the interference substance in a high concentration, and can precisely measure a measurement target without being affected with hemoglobin, and the use of an immunoassay reagent.

### Solution to Problem

As a result of intensive studies directed towards achieving the aforementioned object, the present inventor has found that the pH of a measuring reagent comprising a sulfite is set at pH 7.4 or less, and a reducing substance, such as ascorbic acid or a salt thereof or a thiol compound, is further added to the measuring reagent, so that inhibitory effects on the interference substance can be obtained for a long period of time, and the influence of hemoglobin can be suppressed, thereby completing the present invention.

Specifically, the present invention consists of the following configurations:
(1) An immunoassay method of carrying out an antigen-antibody reaction under suppression of interference reaction of a rheumatoid factor using an immunoassay reagent in the presence of a sulfite, wherein the immunoassay reagent comprises a sulfite and a reducing substance.
(2) The immunoassay method according to the above (1), wherein the concentration of the reducing substance is from 1 mM to 300 mM.
(3) The immunoassay method according to the above (1) or (2), wherein the concentration of the sulfite is from 50 mM to 600 mM.
(4) The immunoassay method according to any one of the above (1) to (3), wherein the pH of the immunoassay reagent comprising the sulfite and the reducing substance is from pH 5.6 to 7.4.
(5) The immunoassay method according to any one of the above (1) to (4), wherein the reducing substance is any one or more reducing substances selected from ascorbic acid or a salt thereof and a thiol compound.
(6) The immunoassay method according to the above (5), wherein the thiol compound is any one or more thiol compounds selected from 2-mercaptoethylamine, 2-dimethylaminoethanethiol, 2-mercaptoethanol, 3-mercaptopropanol, dithiothreitol, cysteine, N-acetylcysteine, reduced glutathione, and thiophenol.
(7) The immunoassay method according to any one of the above (1) to (6), comprising a step of mixing a first reagent comprising the immunoassay reagent with a second reagent comprising an antibody or an antigen reacting with a measurement target, or a carrier particle immobilizing an antibody or an antigen reacting with a measurement target, wherein the measurement target is quantitatively or qualitatively measured.
(8) The immunoassay method according to any one of the above (1) to (7), which is latex agglutination immunoassay.
(9) Use of an immunoassay reagent in an immunoassay method of carrying out an antigen-antibody reaction under suppression of interference reaction of a rheumatoid factor in the presence of a sulfite, wherein the immunoassay reagent comprises a sulfite and a reducing substance.
(10) Use according to the above (9), wherein the concentration of the reducing substance is from 1 mM to 300 mM.
(11) Use according to the above (9) or (10), wherein the concentration of the sulfite is from 50 mM to 600 mM.
(12) Use according to any one of the above (9) to (11), wherein the pH of the immunoassay reagent comprising the sulfite and the reducing substance is from pH 5.6 to 7.4.
(13) Use according to any one of the above (9) to (12), wherein the reducing substance is any one or more reducing substances selected from ascorbic acid or a salt thereof and a thiol compound.
(14) Use according to the above (13), wherein the thiol compound is any one or more thiol compounds selected from 2-mercaptoethylamine, 2-dimethylaminoethanethiol, 2-mercaptoethanol, 3-mercaptopropanol, dithiothreitol, cysteine, N-acetylcysteine, reduced glutathione, and thiophenol.
(15) Use according to any one of the above (9) to (14), wherein the immunoassay method is latex agglutination immunoassay.
(16) Use of an immunoassay reagent kit in an immunoassay method of carrying out an antigen-antibody reaction under suppression of interference reaction of a rheumatoid factor in the presence of a sulfite, comprising a first reagent comprising the immunoassay reagent as defined in any one of the above (9) to (15) and a second reagent comprising an antibody or an antigen reacting with a measurement target, or a carrier particle immobilizing an antibody or an antigen reacting with a measurement target.

### Advantageous Effects of Invention

The present invention provides an immunoassay method and the use of an immunoassay reagent with excellent stability, with which an immunological measurement can be carried out without being affected with an interference substance, even in a case where an interference substance against an antigen-antibody reaction is contained in a high concentration in a specimen that is a measurement target, by allowing an immunoassay reagent with pH 7.4 or less, containing a sulfite, to further comprise a reducing substance, such as ascorbic acid or a salt thereof or a thiol compound. Moreover, the present invention provides an immunoassay and the use of an immunoassay reagent with excellent stability, with which an immunological measurement can be carried out with high precision even in a case where hemoglobin is contained in a specimen that is a measurement target.

It is anticipated that the results obtained by this immunological measurement will be highly reliable.

### Brief Description of Drawings

[Figure 1] Figure 1 shows changes over time in the absorption spectrum of hemoglobin, when the pH of the first reagent is 7.4.
[Figure 2] Figure 2 shows changes over time in the absorption spectrum of hemoglobin, when the pH of the first reagent is 6.7.
[Figure 3] Figure 3 shows changes over time in the absorption spectrum of hemoglobin, when the pH of the first reagent is 6.7 and 10 mM sodium ascorbate is added.
[Figure 4] Figure 4 shows changes overtime in the absorption spectrum of hemoglobin, when the pH of the first reagent is 6.7 and 10 mM 2-mercaptoethylamine is added.
[Figure 5] Figure 5 shows changes over time in the absorption spectrum of hemoglobin, when the pH of the first reagent is 6.7 and 10 mM 2-dimethylaminoethanethiol is added.
[Figure 6] Figure 6 shows changes over time in the absorption spectrum of hemoglobin, when the pH of the first reagent is 6.7 and 5 mM dithiothreitol is added.
[Figure 7] Figure 7 shows changes over time in the absorption spectrum of hemoglobin, when the pH of the first reagent is 6.7 and 10 mM 2-mercaptoethanol is added.
[Figure 8] Figure 8 shows changes over time in the absorption spectrum of hemoglobin, when the pH of the first reagent is 6.7 and 10 mM cysteine is added.
[Figure 9] Figure 9 shows changes over time in the absorption spectrum of hemoglobin, when the pH of the first reagent is 6.7 and 10 mM reduced glutathione is added.
[Figure 10] Figure 10 shows changes over time in the absorption spectrum of hemoglobin, when the pH of the first reagent is 6.7, 10 mM sodium ascorbate is added, and sodium sulfite is removed.
[Figure 11] Figure 11 shows changes over time in the absorption spectrum of hemoglobin, when the pH of the first reagent is 6.7 and 0.01 mM sodium ascorbate is added.
[Figure 12] Figure 12 shows changes over time in the absorption spectrum of hemoglobin, when the pH of the first reagent is 6.7 and 0.1 mM sodium ascorbate is added.
[Figure 13] Figure 13 shows changes over time in the absorption spectrum of hemoglobin, when the pH of the first reagent is 6.7 and 0.1 mM 2-mercaptoethylamine is added.
[Figure 14] Figure 14 shows changes over time in the absorption spectrum of hemoglobin, when the pH of the first reagent is 6.7 and 1 mM 2-mercaptoethylamine is added.

### Description of Embodiments

Hereinafter, preferred embodiments of the present invention will be described.

One embodiment of the present invention relates to an immunoassay method of carrying out an antigen-antibody reaction under suppression of interference reaction of a rheumatoid factor using an immunoassay reagent in the presence of a sulfite, wherein the immunoassay reagent comprises a sulfite and a reducing substance.

In the present disclosure, the "reducing substance" means a substance easily releasing hydrogen ions (protons).

In addition, in the present invention, the "immunoassay method" means a method of determining the presence or absence of a measurement target (detection or qualitative measurement), using a substance (e.g., an antibody or an antigen) specifically binding to the measurement target that is a measurement subject, or a method of measuring (quantifying) the abundance of a measurement target, when the measurement target is present. Examples of the immunoassay method include an RIA method and an ELISA method, which are based on the principle of a sandwich method or a competitive method, and turbidimetric immunoassay (TIA) and latex agglutination immunoassay (LIA), which are based on an immune agglutination reaction method.

The "immunoassay reagent" of the present disclosure is a reagent used in an immunoassay method, in which an antigen-antibody reaction is carried out, and the present immunoassay reagent comprises a sulfite and a reducing substance. Moreover, the immunoassay reagent of the present disclosure can be used as a pre-treatment reagent for a measurement specimen containing a measurement target substance.

The "pre-treatment reagent" of the present disclosure is a reagent used to treat a measurement specimen, before the measurement specimen containing a measurement target substance is measured according to an immunoassay method, and the present pre-treatment reagent comprises a sulfite and a reducing substance. The measurement specimen treated with a pre-treatment reagent can be directly used as a measurement specimen in a known immunoassay method.

The sulfite used in the immunoassay method and uses of the present invention can be selected, as appropriate, from known sulfites. Examples of the sulfite used herein include sodium sulfite, potassium sulfite, calcium sulfite, ammonium sulfite, sodium hydrogen sulfite, and ammonium hydrogen sulfite.

When the sulfite is comprised in the immunoassay reagent or the pre-treatment reagent, the concentration of the sulfite in the immunoassay reagent or the pre-treatment reagent is 50 mM or more, preferably 50 to 600 mM, more preferably 100 to 600 mM, and further preferably 250 mM to 500 mM. When the concentration of the sulfite is less than 50 mM, it cannot sufficiently suppress the influence of an interference substance on an immune reaction. On the other hand, when the concentration of the sulfite exceeds 600 mM, the influence of an interference substance cannot be effectively suppressed, and further, an increase in the viscosity of the solution may give adverse effects on the measurement.

Moreover, the reducing substance used in the immunoassay method and uses of the present invention can also be selected, as appropriate, from known reducing substances. Examples of the reducing substance used herein include ascorbic acid or a salt thereof, and a thiol compound.

The ascorbic acid or a salt thereof can be selected, as appropriate, from known substances. Examples of the ascorbic acid or a salt thereof, which is used herein, include ascorbic acid, sodium ascorbate, potassium ascorbate, and calcium ascorbate.

The thiol compound can also be selected, as appropriate, from known thiol compounds. Examples of the thiol compound include 2-mercaptoethylamine, 2-dimethylaminoethanethiol, 2-mercaptoethanol, 3-mercaptopropanol, dithiothreitol, cysteine, N-acetylcysteine, reduced glutathione, and thiophenol.

When the reducing substance is comprised in the immunoassay reagent or the pre-treatment reagent, the concentration of the reducing substance in the immunoassay reagent or the pre-treatment reagent is 1 mM or more, preferably 1 to 300 mM, more preferably 1 to 100 mM, and further preferably 1 to 50 mM. When the concentration of the reducing substance is 1 mM or more, the influence of hemoglobin can be effectively avoided. However, if the concentration of the reducing substance exceeds 300 mM, it may give adverse effects on the measurement, and a precise measurement may not be carried out in some cases.

The pH of the immunoassay reagent or pre-treatment reagent containing a sulfite and a reducing substance of the present disclosure is preferably pH 7.4 or less, more preferably in the range from pH 5.6 to pH 7.4, and further preferably in the range from pH 6.7 to pH 6.9. When the pH of the present immunoassay reagent or pre-treatment reagent is lower than pH 5.6, a protein and the like may be easily denatured, and it may give influence on a precise measurement. On the other hand, when the pH of the present immunoassay reagent or pre-treatment reagent exceeds pH 7.4, the effect of suppressing the influence of an interference substance may be easily decreased over time.

In the immunoassay method and uses of the present invention, a known protein protecting agent is added, as necessary, so that the stability of a measurement target substance can be enhanced. Examples of such a protein protecting agent include proteins such as albumin or gelatin, synthetic polymer materials, and surfactants.

The specimen, in which the influence of an interference substance has been suppressed by the immunoassay reagent or pre-treatment reagent containing a sulfite and a reducing substance of the present disclosure , can be directly measured according to a known immunoassay method. Examples of the immunoassay method applied herein include an RIA method and an ELISA method, which are based on the principle of a sandwich method or a competitive method, and turbidimetric immunoassay (TIA) and latex agglutination immunoassay (LIA), which are based on an immune agglutination reaction method.

Among these immunoassay methods, turbidimetric immunoassay or latex agglutination immunoassay, which is based on a measurement principle that an antigen (or an antibody) that is a substance to be measured contained in a measurement specimen is allowed to react with an antibody (or an antigen) in a measurement reagent, and the resulting immune aggregate is then detected, is particularly preferable.

The immunoassay reagent of the present disclosure can be used in turbidimetric immunoassay or latex agglutination immunoassay, and the present immunoassay reagent can be composed of two reagents, namely, a first reagent containing a buffer, and a second reagent allowing a buffer to contain an antibody or an antigen, or a carrier particle (preferably, a latex particle) immobilizing an antibody or an antigen. Moreover, these two reagents can also be provided in the form of an immunoassay reagent kit.

The buffer that can be used in the immunoassay reagent for turbidimetric immunoassay or latex agglutination immunoassay is not particularly limited, as long as the pH thereof can be maintained at a constant value. The buffer can be selected, as appropriate, from known buffers. Examples of the buffer used herein include a phosphate buffer, a Tris buffer, and a Good's buffer.

The immunoassay reagent containing a sulfite and a reducing substance of the present disclosure can also be used as a first reagent for immunoassay methods, such as turbidimetric immunoassay or latex agglutination immunoassay, involving the above-described two reagents. That is, a measurement specimen may be mixed with a first reagent, in which the method of suppressing the influence of an interference substance of the present invention is applied, and thereafter, a second reagent containing an antibody (or an antigen) or a latex particle immobilizing an antibody (or an antigen) may be mixed with the above obtained mixed solution, followed by performing a measurement.

Otherwise, a pre-treatment reagent, in which the method of suppressing the influence of an interference substance of the present invention is applied, may be mixed with a measurement specimen, and thereafter, the mixed solution may be used as a measurement specimen in an immunoassay method such as an ELISA method or latex agglutination immunoassay, and a measurement may be then performed.

In the immunoassay method and uses of the present invention, the antibody or the antigen immobilized on a carrier particle is not particularly limited, and it can be selected, as appropriate, from known antibodies or antigens. Examples of the antibody or the antigen immobilized on a carrier particle include: antibodies reacting against plasma proteins such as a C-reactive protein (CRP) or transferrin; antibodies reacting against hormones such as thyroid-stimulating hormone (TSH), thyroxine, insulin, or human placental lactogen; antibodies reacting against tumor-related substances such as carcinoembryonic antigen (CEA), β2-microglobulin, or α-fetoprotein (AFP); antibodies reacting against viral hepatitis antigens such as HBs antigen or HBe antigen; antigens reacting against viral hepatitis antibodies such as HBs antibody or HBe antibody; antibodies or antigens reacting against viruses such as herpes virus, measles virus or rubella virus, or various types of biological components; and antibodies reacting against various types of drugs such as phenobarbital, acetaminophen or cyclosporin.

In the immunoassay method and uses of the present invention, the carrier particle immobilizing an antibody or an antigen is not particularly limited, and it can be selected, as appropriate, from known carrier particles. Examples of the carrier particle immobilizing an antibody or an antigen include a polystyrene plate, a polystyrene bead, a latex particle, a metal colloidal particle, and a silica colloidal particle.

The animal species, from which the above-described antibody that can be used in the immunoassay method and uses of the present invention is derived, is not particularly limited. Examples of the antibody used herein include antibodies derived from animals such as a rabbit, a goat, a mouse, a rat, a horse or sheep. Either a polyclonal antibody obtained from the serum of an animal immunized with a measurement target, or a monoclonal antibody obtained by fusing the spleen of an animal immunized with a measurement target with myeloma cells, may be used.

In the immunoassay method and uses of the present invention, the measurement specimen is not particularly limited, and examples of the measurement specimen include blood, serum, plasma, urine, lymph fluid, puncture fluid, cerebrospinal fluid, sweat, saliva, gastric juice, bronchoalveolar lavage fluid, and feces. Among these specimens, serum and plasma are particularly preferable. According to the immunoassay method and uses of the present invention, when a measurement specimen such as serum or plasma is hemolyzed, the influence of hemoglobin can be avoided, and thus, a precise measurement can be carried out.

In addition, in the immunoassay method and uses of the present invention, the measurement target substance is not particularly limited, either. Examples of the measurement target substance include: plasma proteins such as a C-reactive protein (CRP) or transferrin; hormones such as thyroid-stimulating hormone (TSH), thyroxine, insulin, or human placental lactogen; tumor-related substances such as carcinoembryonic antigen (CEA), β2-microglobulin, or α-fetoprotein (AFP); viral hepatitis antigens such as HBs antigen or HBe antigen; viral hepatitis antibodies such as HBs antibody or HBe antibody; antibodies or antigens reacting against viruses such as herpes virus, measles virus or rubella virus, or various types of biological components; and various types of drugs such as phenobarbital, acetaminophen or cyclosporin.

### Examples

Hereinafter, the present invention will be specifically described in the following examples. Example 1 Influence of sulfite concentration on effect of suppressing interference reaction

The influence of the concentration of a sulfite on the suppression of an interference reaction was evaluated by addition of sodium sulfite. In addition, a sulfite was stored under conditions for promoting oxidation of the sulfite (i.e., the sulfite was filled into a half of a reagent vessel and was then shaken at 4°C for 3 weeks), and the stability was then evaluated.

### (1) Reagents

As a first reagent, a 50 mM HEPES buffer (pH 7.4) containing sodium sulfite in a concentration of 0 mM, 50 mM, 100 mM, 250 mM, 350 mM, 500 mM or 600 mM was prepared. Thereafter, the prepared first reagent was filled into a half of a reagent vessel, and was then shaken at 4°C for 3 weeks, so that the oxidation of the sulfite by oxygen contained in the air was promoted.

In addition, as a second reagent, a 50 mM HEPES buffer (pH 7.4) containing an anti-KL-6 antibody-immobilizing polystyrene latex solution was prepared.

The above-described anti-KL-6 antibody-immobilizing latex solution was prepared according to a known method. Specifically, the anti-KL-6 antibody-immobilizing latex solution was prepared by mixing an anti-KL-6 antibody with polystyrene latex, and thus adsorbing the anti-KL-6 antibody on the surface of the polystyrene latex.

### (2) Measurement specimens

Interference Check RF Plus (Sysmex Corporation) was added to pooled serum, so that the concentration of a rheumatoid factor (RF) became 0, 100, 200, 300, 400, 500, or 1000 IU/mL, so as to prepare RF specimens.

### (3) Evaluation method

120 µL of the first reagent was mixed with 2.0 µL of the RF specimen, and the obtained mixture was then incubated at 37°C for 5 minutes. Thereafter, 40 µL of the second reagent was mixed with the above-obtained mixed solution, and the thus obtained mixture was reacted at 37°C. Thereafter, changes in the absorbance at 660 nm were measured for approximately 5 minutes. It is to be noted that a series of measurements were carried out by using Hitachi 7180 Automatic Analyzer (Hitachi High-Technologies Corporation).

In order to quantify the concentration of KL-6, the concentration of KL-6 in the serum specimen was calculated from a calibration curve obtained by the measurement of a standard substance of KL-6. The results obtained immediately after preparation are shown in Table 1, whereas the results obtained after promotion of the oxidation of the sulfite are shown in Table 2.

**[Table 1]**

| | | Concentration of sodium sulfite | | | | | | |
|---|---|---|---|---|---|---|---|---|
| | | 0mM | 50mM | 100mM | 250mM | 350mM | 500mM | 600mM |
| Measurement value of KL-6 (U/mL) | | | | | | | | |
| RF IU/mL | 0 | 1211 | 1204 | 1219 | 1182 | 1179 | 1181 | 1193 |
| | 100 | 1262 | 1238 | 1251 | 1207 | 1204 | 1213 | 1190 |
| | 200 | 1371 | 1302 | 1297 | 1234 | 1233 | 1242 | 1226 |
| | 300 | 1376 | 1305 | 1297 | 1238 | 1223 | 1223 | 1222 |
| | 400 | 1491 | 1353 | 1336 | 1253 | 1227 | 1243 | 1262 |
| | 500 | 1489 | 1362 | 1343 | 1261 | 1252 | 1254 | 1262 |
| | 1000 | 1874 | 1527 | 1443 | 1285 | 1262 | 1285 | 1320 |

| Recovery percentage (%)* | | | | | | | | |
|---|---|---|---|---|---|---|---|---|
| RF IU/mL | 0 | 100% | 100% | 100% | 100% | 100% | 100% | 100% |
| | 100 | 104% | 103% | 103% | 102% | 102% | 103% | 100% |
| | 200 | 113% | 108% | 106% | 104% | 105% | 105% | 103% |
| | 300 | 114% | 108% | 106% | 105% | 104% | 104% | 102% |
| | 400 | 123% | 112% | 110% | 106% | 104% | 105% | 106% |
| | 500 | 123% | 113% | 110% | 107% | 106% | 106% | 106% |
| | 1000 | 155% | 127% | 118% | 109% | 107% | 109% | 111% |

| | | | | | | | | |
|---|---|---|---|---|---|---|---|---|
| *: Value obtained when non-addition of RF (0 IU/mL) is set at 100% | | | | | | | | |

**[Table 2]**

| | | Concentration of sodium sulfite | | | | | | |
|---|---|---|---|---|---|---|---|---|
| | | 0mM | 50mM | 100mM | 250mM | 350mM | 500mM | 600mM |
| Measurement value of KL-6 (U/mL) | | | | | | | | |
| RF IU/mL | 0 | 1207 | 1216 | 1215 | 1182 | 1162 | 1170 | 1173 |
| | 100 | 1260 | 1274 | 1254 | 1209 | 1192 | 1188 | 1191 |
| | 200 | 1370 | 1361 | 1295 | 1234 | 1211 | 1211 | 1227 |
| | 300 | 1358 | 1358 | 1297 | 1239 | 1210 | 1213 | 1261 |
| | 400 | 1487 | 1452 | 1347 | 1253 | 1222 | 1232 | 1276 |
| | 500 | 1517 | 1455 | 1345 | 1264 | 1234 | 1240 | 1268 |
| | 1000 | 1846 | 1744 | 1500 | 1312 | 1275 | 1282 | 1363 |

| Recovery percentage (%)* | | | | | | | | |
|---|---|---|---|---|---|---|---|---|
| RF IU/mL | 0 | 100% | 100% | 100% | 100% | 100% | 100% | 100% |
| | 100 | 104% | 105% | 103% | 102% | 103% | 102% | 101% |
| | 200 | 114% | 112% | 107% | 104% | 104% | 104% | 105% |
| | 300 | 113% | 112% | 107% | 105% | 104% | 104% | 107% |
| | 400 | 123% | 119% | 111% | 106% | 105% | 105% | 109% |
| | 500 | 126% | 120% | 111% | 107% | 106% | 106% | 108% |
| | 1000 | 153% | 143% | 124% | 111% | 110% | 110% | 116% |

| | | | | | | | | |
|---|---|---|---|---|---|---|---|---|
| *: Value obtained when non-addition of RF (0 IU/mL) is set at 100% | | | | | | | | |

From Table 1, it was found that the effect of suppressing an interference reaction caused by RF was observed at a sulfite concentration of 50 mM or more, and that, even at a sulfite concentration of 50 mM, the interference reaction caused by RF could be decreased to approximately one half of the case of non-addition of the sulfite (0 mM). Moreover, it was also found that the effect of suppressing the interference reaction was improved depending on the sulfite concentration, and that the suppressive effect became maximum at a sulfite concentration of 250 mM to 500 mM, and was decreased at a sulfite concentration of 600 mM. The sulfite concentration is preferably 50 mM or more, more preferably 100 mM to 600 mM, and most preferably 250 to 500 mM.

Furthermore, as shown in Table 1 and Table 2, when the sulfite concentration was 50 mM, the effect of suppressing the interference reaction was decreased as a result of the oxidation of the sulfite. It was found that the influence of the oxidation of the sulfite on the effect of suppressing the interference reaction was decreased depending on the sulfite concentration, and the influence became minimum at a sulfite concentration of 250 mM to 500 mM, so that the stability was improved, but that the effect of suppressing the interference reaction was decreased as a result of the oxidation of the sulfite, when the concentration of the sulfurous acid was 600 mM.

### Example 2 Influence of pH on suppression of interference reaction by sulfite

Sodium sulfite was used at a concentration of 250 mM, and the influence of pH on suppression of the interference reaction caused by an interference substance was evaluated.

### (1) Reagents

As a first reagent, a 50 mM PIPES buffer (pH 6.4 or 6.8) or a 50 mM HEPES buffer (pH 7.4, 7.8 or 8.2), each containing 250 mM sodium sulfite, was prepared. Regarding a second reagent, the same second reagent as that used in Example 1 was used.

### (2) Measurement specimens

Interference Check RF Plus (Sysmex Corporation) was added to pooled serum, so that the concentration of a rheumatoid factor (RF) became 0, 100, 200, 300, 400, or 500 IU/mL, thereby preparing RF specimens. In addition to the RF specimens, normal specimens (heterophile antibody and RF-negative human serum specimens) and abnormal specimens (heterophile antibody and RF-positive human serum specimens) were used as measurement specimens.

### (3) Evaluation method

The specimens were measured in the same manner as that of Example 1. The results of the RF specimens are shown in Table 3, and the results of the human serum specimens are shown in Table 4.

**[Table 3]**

| | | pH | | | | |
|---|---|---|---|---|---|---|
| | | 6.4 | 6.8 | 7.4 | 7.8 | 8.2 |
| Measuremen t value of KL-6 (U/mL) | | | | | | |
| RF IU/mL | 0 | 419 | 453 | 465 | 446 | 455 |
| | 100 | 421 | 453 | 466 | 463 | 486 |
| | 200 | 441 | 463 | 474 | 489 | 525 |
| | 300 | 433 | 455 | 487 | 509 | 551 |
| | 400 | 443 | 468 | 486 | 511 | 579 |
| | 500 | 455 | 468 | 488 | 517 | 609 |

| Recovery per centage (%)* | | | | | | |
|---|---|---|---|---|---|---|
| RF IU/mL | 0 | 100% | 100% | 100% | 100% | 100% |
| | 100 | 100% | 100% | 100% | 104% | 107% |
| | 200 | 105% | 102% | 102% | 110% | 115% |
| | 300 | 103% | 101% | 105% | 114% | 121% |
| | 400 | 106% | 103% | 104% | 115% | 127% |
| | 500 | 109% | 103% | 105% | 116% | 134% |

| | | | | | | |
|---|---|---|---|---|---|---|
| *: Value obtained when non-addition of RF (0 IU/mL) is set at 100% | | | | | | |

**[Table 4]**

| | | pH | | | | |
|---|---|---|---|---|---|---|
| | | 6.4 | 6.8 | 7.4 | 7.8 | 8.2 |
| Measurement val ue of K L-6 (U/m L) | | | | | | |
| Normal specimen | 1 | 190 | 204 | 209 | 146 | 193 |
| | 2 | 315 | 333 | 330 | 313 | 324 |
| | 3 | 428 | 456 | 460 | 444 | 439 |
| | 4 | 365 | 386 | 387 | 369 | 375 |
| | 5 | 598 | 620 | 629 | 622 | 632 |
| | 6 | 1356 | 1434 | 1434 | 1470 | 1412 |
| Abnormal specimen | 1 | 552 | 582 | 615 | 616 | 608 |
| | 2 | 507 | 517 | 546 | 528 | 534 |
| | 3 | 432 | 501 | 563 | 717 | 1069 |
| | 4 | 580 | 669 | 831 | 1579 | 4958 |

| Recovery percent age (% )* | | | | | | |
|---|---|---|---|---|---|---|
| Normal specimen | 1 | 93% | 100% | 102% | 71% | 95% |
| | 2 | 95% | 100% | 99% | 94% | 97% |
| | 3 | 94% | 100% | 101% | 97% | 96% |
| | 4 | 95% | 100% | 100% | 96% | 97% |
| | 5 | 97% | 100% | 102% | 100% | 102% |
| | 6 | 95% | 100% | 100% | 103% | 98% |
| Abnormal specimen | 1 | 95% | 100% | 106% | 106% | 105% |
| | 2 | 98% | 100% | 106% | 102% | 103% |
| | 3 | 86% | 100% | 112% | 143% | 214% |
| | 4 | 87% | 100% | 124% | 236% | 741% |

| | | | | | | |
|---|---|---|---|---|---|---|
| *: Value obtained when pH 6.8 is set at 100% | | | | | | |

As is clear from Table 3, the effect of the sulfite to suppress the interference reaction of RF was significantly decreased at a pH value of 7.8 or more, as the pH value increased, and the positive error of the measurement value increased. However, at a pH value of 7.4 or less, a large difference was not found in the effect of the sulfite to suppress the interference reaction of RF.

In addition, as shown in Table 4, the influence of the pH on the suppression of the interference reaction by the sulfite is hardly observed in the normal specimens. In contrast, in the abnormal specimens in which the interference substance is present, as the pH increases, the effect of the sulfite to suppress the interference reaction is significantly decreased, and the measurement value is increased. However, a fluctuation in the measurement values is small at pH 6.4 and pH 6.8, and thus, it is found that the interference reaction is effectively suppressed.

### Example 3 Influence of pH of sulfite-containing reagent on influence of hemoglobin

The concentration of sodium sulfite was set at 250 mM, and the influence of pH on the influence of hemoglobin (Hb) and suppression of the interference reaction of a rheumatoid factor was evaluated.

### (1) Reagents

As a first reagent, a 50 mM PIPES buffer (pH 6.7) or a 50 mM HEPES buffer (pH 7.4), each containing 250 mM sodium sulfite, was prepared. Regarding a second reagent, the same second reagent as that used in Example 1 was used.

### (2) Measurement specimens

Interference Check RF Plus (Sysmex Corporation) was added to pooled serum, so that the concentration of a rheumatoid factor (RF) became 0, 100, 200, 300, 400, 500 or 1000 IU/mL, thereby preparing RF specimens. At the same time, Interference Check A Plus hemolyzed hemoglobin (Sysmex Corporation) was added to pooled serum, so that the concentration of hemoglobin became 0, 100, 200, 300, 400, 500, 600, 800 or 1000 mg/dL, thereby preparing Hb specimens.

### (3) Evaluation method

The specimens were measured in the same manner as that of Example 1. The results are shown in Table 5.

**[Table 5]**

| pH | | Influence of RF | | Influence of Hb | |
|---|---|---|---|---|---|
| | | 7.4 | 6.7 | 7.4 | 6.7 |
| Measurement value of KL-6 (U/mL) mL) | | | | | |
| Hb mg/dL or RF IU/mL | 0 | 1174 | 1148 | 465 | 443 |
| | 100 | 1192 | 1169 | 463 | 457 |
| | 200 | 1225 | 1173 | 459 | 465 |
| | 300 | 1240 | 1185 | 461 | 476 |
| | 400 | 1251 | 1167 | 459 | 485 |
| | 500 | 1266 | 1179 | 459 | 496 |
| | 600 | - | - | 457 | 517 |
| | 800 | - | - | 458 | 550 |
| | 1000 | 1303 | 1169 | 458 | 578 |

| Recovery perc entage (%)* | | | | | |
|---|---|---|---|---|---|
| Hb mg/dL or RF IU/mL | 0 | 100% | 100% | 100% | 100% |
| | 100 | 102% | 102% | 100% | 103% |
| | 200 | 104% | 102% | 99% | 105% |
| | 300 | 106% | 103% | 99% | 107% |
| | 400 | 107% | 102% | 99% | 109% |
| | 500 | 108% | 103% | 99% | 112% |
| | 600 | - | - | 98% | 117% |
| | 800 | - | - | 99% | 124% |
| | 1000 | 111% | 102% | 99% | 130% |

| | | | | | |
|---|---|---|---|---|---|
| *: Value obtained when non-addition of Hb (0 mg/dL) or non-addition of RF (0 IU/mL) is set at 100% | | | | | |

As is clear from Table 5, when a reagent containing a sulfite had pH 7.4, the interference reaction caused by RF was observed, whereas when it had pH 6.7, the interference reaction caused by RF was suppressed. In contrast, the influence of hemoglobin on measurement values was not observed at pH 7.4, whereas the positive influence was observed depending on the concentration of hemoglobin at pH 6.7.

### Example 4 Confirmation of cause of influence of hemoglobin

The concentration of sodium sulfite was set at 250 mM, and the influence of pH on the absorption spectrum of hemoglobin was then evaluated.

### (1) Reagents

Regarding a first reagent, the same first reagent as that used in Example 3 was used. A second reagent was prepared by eliminating anti-KL-6 antibody-immobilizing latex from the composition of the second reagent of Example 1.

### (2) Measurement specimens

Interference Check A Plus hemolyzed hemoglobin (Sysmex Corporation) was added to pooled serum to a hemoglobin concentration of 500 mg/dL, thereby preparing a Hb specimen.

### (3) Evaluation method

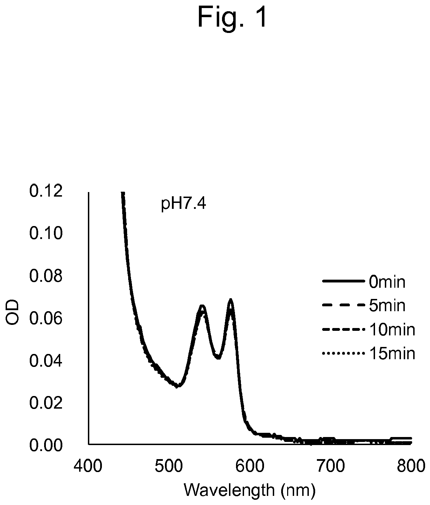

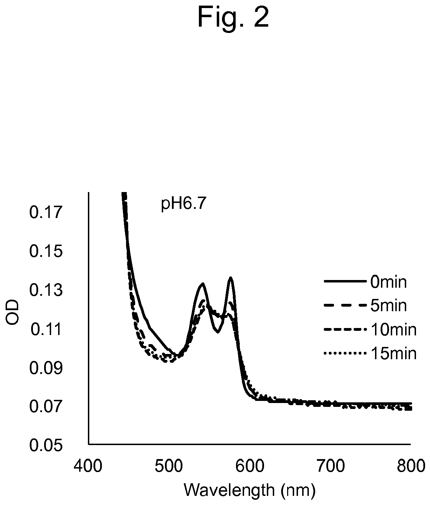

600 µL of the first reagent was added to 10 µL of the Hb specimen, and the second reagent was then added to this mixture. Thereafter, the absorption spectra were measured at wavelengths from 800 nm to 400 nm, using SHIMADZU UV-2600 (Shimadzu Corporation). After completion of the measurement of absorption spectra, the mixed solution was heated at 37°C, and 5 minutes after initiation of the heating, the absorption spectra were measured at wavelengths from 800 nm to 400 nm, and after that, the absorption spectra were measured at wavelengths from 800 nm to 400 nm every 5 minutes. The results are shown in Figure 1 and Figure 2.

As is clear from Figure 1, the pH of the first reagent comprising a sulfite was pH 7.4, changes over time were not observed in the absorption spectrum of hemoglobin. In contrast, it was found that, as in Figure 2, the pH of the first reagent comprising a sulfite was pH 6.7, the absorption spectrum of hemoglobin was changed over time. Such changes over time in the absorption spectrum of hemoglobin have an influence on the measurement values obtained by immunoassay methods that do not involve B/F separation, such as turbidimetric immunoassay or latex agglutination immunoassay, and demonstrate that the cause of the influence on the measurement values is hemoglobin.

### Example 5 Effects of reducing substance on influence of hemoglobin

The concentration of sodium sulfite was set at 250 mM, and pH was set at pH 6.7. Then, the effects of reducing substances such as sodium ascorbate (AA), 2-mercaptoethylamine (2-MEA), 2-dimethylaminoethanethiol (2-DAET), and dithiothreitol (DTT) were evaluated.

### (1) Reagents

As a first reagent, a 50 mM PIPES buffer (pH 6.7) containing 250 mM sodium sulfite and a 10 mM reducing substance (DTT: 5 mM) was prepared. In addition, as a comparative example, a 50 mM PIPES buffer that did not contain a reducing substance was also prepared. Moreover, regarding sodium ascorbate and 2-mercaptoethylamine, a 50 mM PIPES buffer, from which sodium sulfite was eliminated, was also prepared.

Regarding a second reagent, the same second reagent as that used in Example 1 was used.

### (2) Measurement specimens

Measurement specimens were prepared in the same manner as that of Example 3.

### (3) Evaluation method

The specimens were measured in the same manner as that of Example 1. The results are shown in Table 6 and Table 7.

**[Table 6]**

| Concentration of Na sulfite | | 250mM | | | | | Not added | |
|---|---|---|---|---|---|---|---|---|
| Reducing substance | | Comp. Ex. | AA | 2-MEA | 2-DAET | DTT | AA | 2-MEA |
| Concentration of reducing substance | | | 10mM | 10mM | 10mM | 5mM | 10mM | 10mM |
| pH | | 6.7 | | | | | | |
| :Measurement value of KL-6 (U/mL) | | | | | | | | |
| RF IU/mL | 0 | 1243 | 1221 | 1236 | 1242 | 1227 | 1227 | 1237 |
| | 100 | 1206 | 1230 | 1197 | 1206 | 1194 | 1253 | |
| | 200 | 1210 | 1233 | 1196 | 1210 | 1197 | 1280 | 1318 |
| | 300 | 1215 | 1232 | 1202 | 1206 | 1204 | 1306 | |
| | 400 | 1209 | 1237 | 1201 | 1210 | 1202 | 1329 | |
| | 500 | 1224 | 1245 | 1208 | 1214 | 1212 | 1368 | 1399 |
| | 1000 | 1201 | 1252 | 1192 | 1199 | 1194 | 1496 | |

| Recovery percentage (%)* | | | | | | | | |
|---|---|---|---|---|---|---|---|---|
| RF IU/mL | 0 | 100% | 100% | 100% | 100% | 100% | 100% | 100% |
| | 100 | 97% | 101% | 97% | 97% | 97% | 102% | |
| | 200 | 97% | 101% | 97% | 97% | 98% | 104% | 107% |
| | 300 | 98% | 101% | 97% | 97% | 98% | 106% | |
| | 400 | 97% | 101% | 97% | 97% | 98% | 108% | |
| | 500 | 99% | 102% | 98% | 98% | 99% | 111% | 113% |
| | 1000 | 97% | 103% | 96% | 97% | 97% | 122% | |

| | | | | | | | | |
|---|---|---|---|---|---|---|---|---|
| *: Value obtained when non-addition of RF (0 IU/mL) is set at 100% | | | | | | | | |

**[Table 7]**

| Concentration of Na sulfite | | 250mM | | | | | Not added | |
|---|---|---|---|---|---|---|---|---|
| Reducing substance | | Comp. Ex. | AA | 2-MEA | 2-DAET | DTT | AA | 2-MEA |
| Concentration of reducing substance | | | 10mM | 10mM | 10mM | 5mM | 10mM | 10mM |
| pH | | 6.7 | | | | | | |
| Measurement value of KL-6 (U/mL) | | | | | | | | |
| Hb mg/dL | 0 | 443 | 458 | 451 | 453 | 447 | 489 | 443 |
| | 100 | 457 | 456 | 453 | 451 | 449 | 506 | |
| | 200 | 465 | 461 | 449 | 452 | 445 | 517 | 450 |
| | 300 | 476 | 461 | 451 | 454 | 450 | 515 | |
| | 400 | 485 | 457 | 448 | 452 | 448 | 518 | |
| | 500 | 496 | 456 | 446 | 450 | 449 | 526 | 453 |
| | 600 | 517 | 438 | 442 | 449 | 449 | 519 | |
| | 800 | 550 | 447 | 449 | 452 | 444 | 527 | |
| | 1000 | 578 | 451 | 445 | 454 | 446 | 536 | |

| Recovery percentage (%)* | | | | | | | | |
|---|---|---|---|---|---|---|---|---|
| Hb mg/dL | 0 | 100% | 100% | 100% | 100% | 100% | 100% | 100% |
| | 100 | 103% | 100% | 100% | 100% | 100% | 103% | |
| | 200 | 105% | 101% | 100% | 100% | 100% | 106% | 102% |
| | 300 | 107% | 101% | 100% | 100% | 101% | 105% | |
| | 400 | 109% | 100% | 99% | 100% | 100% | 106% | |
| | 500 | 112% | 100% | 99% | 99% | 100% | 108% | 102% |
| | 600 | 117% | 96% | 98% | 99% | 100% | 106% | |
| | 800 | 124% | 98% | 100% | 100% | 99% | 108% | |
| | 1000 | 130% | 99% | 99% | 100% | 100% | 110% | |

| | | | | | | | | |
|---|---|---|---|---|---|---|---|---|
| *: Value obtained when non-addition of Hb (0 mg/dL) is set at 100% | | | | | | | | |

As is clear from Table 6, even if sodium ascorbate, 2-mercaptoethylamine, 2-dimethylaminoethanethiol, or dithiothreitol was added to the first reagent containing sodium sulfite, the interference reaction caused by RF was not observed, as in the case of the comparative example. In contrast, when sodium ascorbate or 2-mercaptoethylamine was used alone without using sodium sulfite, the interference reaction caused by RF was observed.

As is clear from Table 7, it was found that the comparative example was significantly influenced by hemoglobin, whereas the influence of hemoglobin was suppressed by addition of a reducing substance such as sodium ascorbate, 2-mercaptoethylamine, 2-dimethylaminoethanethiol or dithiothreitol. In the case of the single use of sodium ascorbate, the influence of hemoglobin was observed. However, surprisingly, the influence of hemoglobin could be suppressed by combining sodium ascorbate with a sulfite (comparative example), regarding which the influence of hemoglobin was also observed by the single use of the sulfite.

### Example 6 Confirmation of effect of adding reducing substance

The concentration of sodium sulfite was set at 250 mM, and the pH was set at pH 6.7. Then, the effects of a reducing substance that was sodium ascorbate (AA), 2-mercaptoethylamine (2-MEA), 2-dimethylaminoethanethiol (2-DAET), dithiothreitol (DTT), 2-mercaptoethanol (2-ME), cysteine, or reduced glutathione on the absorption spectrum of hemoglobin were evaluated.

### (Reagents)

As first reagents, buffers to which 2-mercaptoethanol, cysteine or reduced glutathione was added in each concentration of 10 mM were prepared, as well as the first reagent of Example 5. As a second reagent, the same second reagent as that of Example 4 was used.

### (2) Measurement specimens

Measurement specimens were prepared in the same manner as that of Example 4.

### (3) Evaluation method

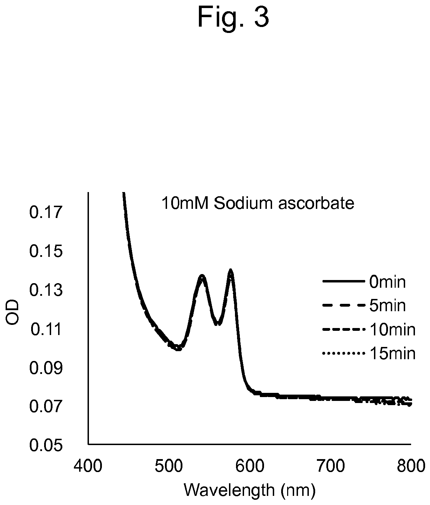

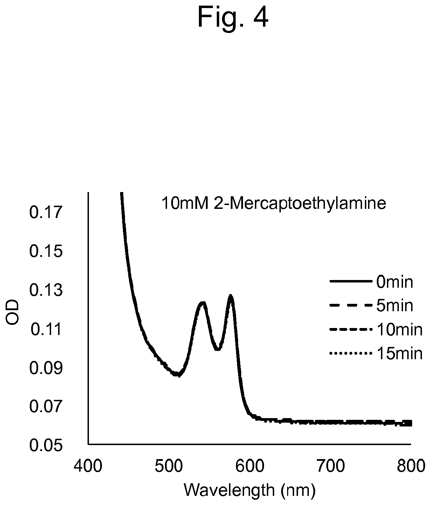

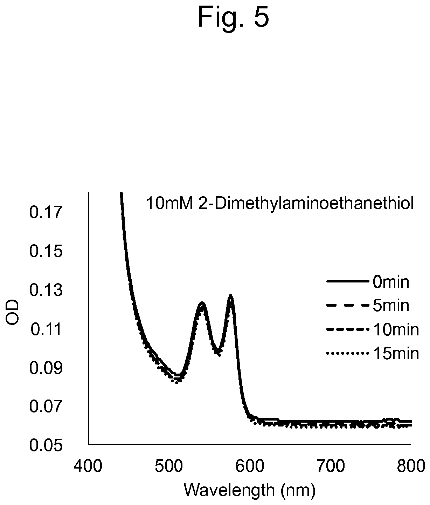

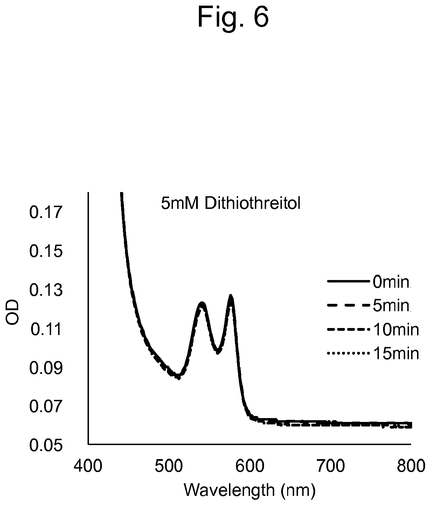

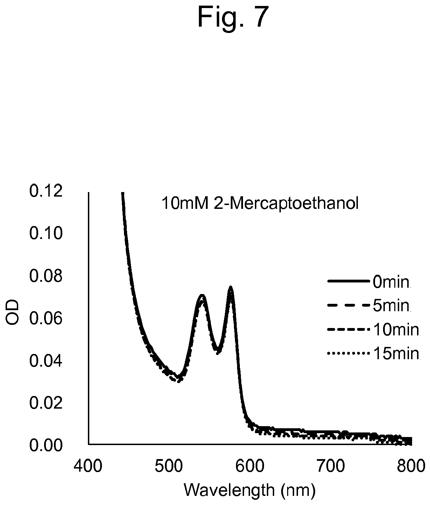

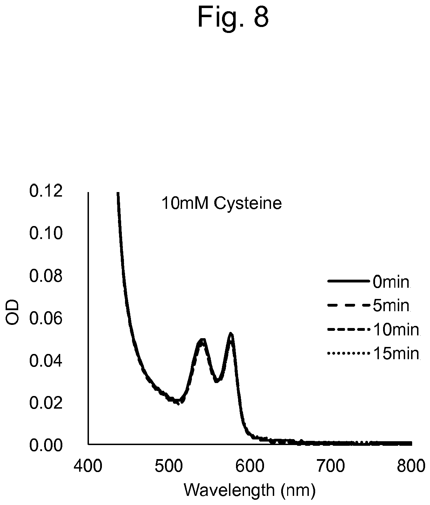

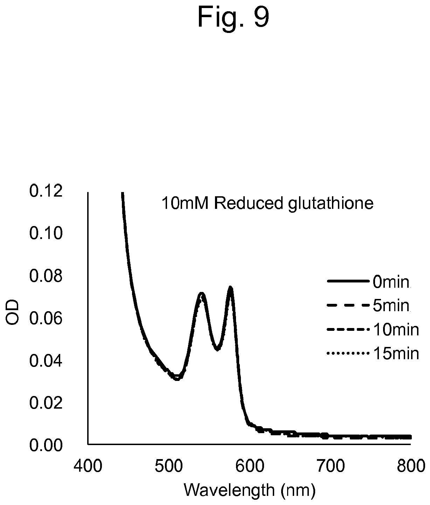

Changes over time in the absorption spectrum of hemoglobin were measured in the same manner as that of Example 4. The results are shown in Figure 3 to Figure 10.

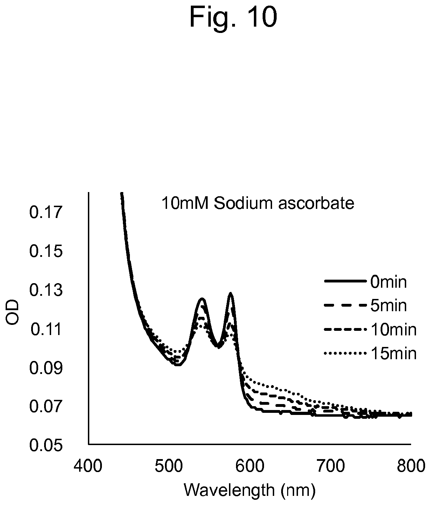

As is clear from Figure 3 to Figure 9, in a case where the first reagent contained a reducing substance such as sodium ascorbate, 2-mercaptoethylamine, 2-dimethylaminoethanethiol, dithiothreitol, 2-mercaptoethanol, cysteine or reduced glutathione, in addition to sodium sulfite, the absorption spectrum of hemoglobin was not changed over time and thus, was stable. In contrast, when sodium ascorbate was added alone, while removing sodium sulfite (Figure 10), the absorption spectrum of hemoglobin was changed over time.

### Example 7 Influence of pH in case of addition of sulfite and 2-mercaptoethylamine

Sodium sulfite was used at 250 mM, and 2-mercaptoethylamine was used at 10 mM Then, the influence of pH on the interference reaction of a rheumatoid factor or the influence of hemoglobin was evaluated.

### (1) Reagents

As a first reagent, a 50 mM PIPES buffer (pH 6.0, 6.2, 6.7 or 6.9) or a 50 mM HEPES buffer (pH 7.4 or 8.0), each containing 250 mM sodium sulfite and 10 mM 2-mercaptoethylamine, was prepared. Regarding a second reagent, the same second reagent as that used in Example 1 was used.

### (2) Measurement specimens

Interference Check RF Plus (Sysmex Corporation) was added to pooled serum, so that the concentration of a rheumatoid factor became 0, 250 or 500 IU/mL, thereby preparing RF specimens. At the same time, Interference Check A Plus hemolyzed hemoglobin (Sysmex Corporation) was added to pooled serum, so that the concentration of hemoglobin became 0, 250 or 500 mg/dL, thereby preparing Hb specimens.

### (3) Evaluation method

The specimens were measured in the same manner as that of Example 1. The results are shown in Table 8 and Table 9.

**[Table 8]**

| | | | | | | | | |
|---|---|---|---|---|---|---|---|---|
| | pH | 6.0 | 6.2 | 6.4 | 6.7 | 6.9 | 7.4 | 8.0 |

| Measuremen t value of KL-6 (U/mL) | | | | | | | | |
|---|---|---|---|---|---|---|---|---|
| RF IU/mL | 0 | 1211 | 1197 | 1193 | 1185 | 1181 | 1201 | 1255 |
| | 250 | 1219 | 1196 | 1196 | 1196 | 1192 | 1213 | 1339 |
| | 500 | 1222 | 1200 | 1211 | 1213 | 1218 | 1248 | 1418 |

| Recovery per centage (%)* | | | | | | | | |
|---|---|---|---|---|---|---|---|---|
| RF IU/mL | 0 | 100% | 100% | 100% | 100% | 100% | 100% | 100% |
| | 250 | 101% | 100% | 100% | 101% | 101% | 101% | 107% |
| | 500 | 101% | 100% | 101% | 102% | 103% | 104% | 113% |
| *: Value obtai ned whe n non-a ddition o f RF (0 IU/mL) is set at 100% | | | | | | | | |

**[Table 9]**

| | | | | | | | | |
|---|---|---|---|---|---|---|---|---|
| pH | | 6.0 | 6.2 | 6.4 | 6.7 | 6.9 | 7.4 | 8.0 |
| Measuremen t value of KL-6 (U/mL) | | | | | | | | |
| Hb mg/dL | 0 | 384 | 364 | 368 | 374 | 390 | 397 | 395 |
| | 250 | 384 | 363 | 375 | 374 | 386 | 396 | 389 |
| | 500 | 380 | 361 | 368 | 369 | 386 | 395 | 390 |
| Recovery per centage (%)* | | | | | | | | |
| Hb mg/dL | 0 | 100% | 100% | 100% | 100% | 100% | 100% | 100% |
| | 250 | 100% | 100% | 102% | 100% | 99% | 100% | 99% |
| | 500 | 99% | 99% | 100% | 99% | 99% | 99% | 99% |

| | | | | | | | | |
|---|---|---|---|---|---|---|---|---|
| *: Value obtained when non-addition of Hb (0 mg/dL) is set at 100% | | | | | | | | |

As is clear from Table 8, at a pH value from pH 6.0 to 7.4, the interference reaction of the rheumatoid factor was suppressed. In contrast, at pH 8.0, the interference reaction of the rheumatoid factor was observed depending on the RF concentration, and the effect of the sulfite to suppress the interference reaction was decreased.

Moreover, as is clear from Table 9, by adding 2-mercaptoethylamine to the sulfite, the influence of hemoglobin was suppressed in the range from pH 6.0 to pH 8.0. The pH of an immunoassay reagent prepared by adding 2-mercaptoethylamine into a sulfite is preferably pH 7.4 or less.

### Example 8 Influence of pH in case of addition of sulfite and ascorbate

Sodium sulfite was used at 250 mM, and sodium ascorbate was used at 10 mM. Then, the influence of pH on the interference reaction of a rheumatoid factor and the influence of hemoglobin was evaluated. In addition, the reagent was stored under conditions of suppressing the oxidation of a sulfite or ascorbate (i.e., the reagent was filled in a reagent vessel as a whole and was left at rest at 4°C) and under conditions of promoting the oxidation thereof (i.e., the reagent was filled in a half of a reagent vessel and was shaking at 4°C for 3 weeks), and the influence of the oxidation of the sulfite or ascorbate was then evaluated.

### (1) Reagents

As a first reagent, a 50 mM PIPES buffer (pH 5.6, 6.0, 6.2, 6.4, 6.7 or 6.9) or a 50 mM HEPES buffer (pH 7.2 or 7.4), each containing 250 mM sodium sulfite and 10 mM sodium ascorbate, was prepared. Regarding a second reagent, the same second reagent as that used in Example 1 was used.

### (2) Measurement specimens

Interference Check RF Plus (Sysmex Corporation) was added to pooled serum, so that the concentration of a rheumatoid factor became 0, 100, 200, 300, 400 or 500 IU/mL, thereby preparing RF specimens. At the same time, Interference Check A Plus hemolyzed hemoglobin (Sysmex Corporation) was added to pooled serum, so that the concentration of hemoglobin became 0, 100, 200, 300, 400 or 500mg/dL, thereby preparing Hb specimens.

### (3) Evaluation method

The specimens were measured in the same manner as that of Example 1. The results are shown in Table 10 and Table 11.

**[Table 10]**

| Storage conditions | | Filling in whole vessel/leaving at rest | | | | | | | | Filling in half of vessel/shaking | | | | | | | |
|---|---|---|---|---|---|---|---|---|---|---|---|---|---|---|---|---|---|
| | pH | 5.6 | 6.0 | 6.2 | 6.4 | 6.7 | 6.9 | 7.2 | 7.4 | 5.6 | 6.0 | 6.2 | 6.4 | 6.7 | 6.9 | 7.2 | 7.4 |
| KL-6(U/mL) | | | | | | | | | | | | | | | | | |
| RF IU/mL | 0 | 868 | 877 | 899 | 881 | 935 | 945 | 955 | 957 | 817 | 881 | 903 | 928 | 955 | 906 | 953 | 949 |
| | 100 | 855 | 887 | 882 | 875 | 931 | 941 | 956 | 957 | 818 | 887 | 902 | 922 | 943 | 923 | 980 | 960 |
| | 200 | 865 | 882 | 890 | 896 | 926 | 937 | 969 | 998 | 839 | 910 | 895 | 929 | 953 | 949 | 987 | 996 |
| | 300 | 874 | 890 | 894 | 896 | 954 | 966 | 979 | 1020 | 849 | 916 | 908 | 934 | 952 | 961 | 997 | 1001 |
| | 400 | 880 | 886 | 879 | 894 | 956 | 967 | 1005 | 1031 | 852 | 920 | 894 | 919 | 959 | 966 | 1022 | 1034 |
| | 500 | 888 | 881 | 885 | 913 | 948 | 966 | 1016 | 1037 | 841 | 922 | 886 | 919 | 946 | 972 | 1031 | 1046 |

| Recovery per centage (%)* | | | | | | | | | | | | | | | | | |
|---|---|---|---|---|---|---|---|---|---|---|---|---|---|---|---|---|---|
| RF IU/mL | 0 | 100% | 100% | 100% | 100% | 100% | 100% | 100% | 100% | 100% | 100% | 100% | 100% | 100% | 100% | 100% | 100% |
| | 100 | 99% | 101% | 98% | 99% | 100% | 100% | 100% | 100% | 100% | 101% | 100% | 99% | 99% | 102% | 103% | 101% |
| | 200 | 100% | 101% | 99% | 102% | 99% | 99% | 101% | 104% | 103% | 103% | 99% | 100% | 100% | 105% | 104% | 105% |
| | 300 | 101% | 102% | 99% | 102% | 102% | 102% | 103% | 107% | 104% | 104% | 101% | 101% | 100% | 106% | 105% | 106% |
| | 400 | 101% | 101% | 98% | 101% | 102% | 102% | 105% | 108% | 104% | 104% | 99% | 99% | 100% | 107% | 107% | 109% |
| | 500 | 102% | 101% | 98% | 104% | 101% | 102% | 106% | 108% | 103% | 105% | 98% | 99% | 99% | 107% | 108% | 110% |

| | | | | | | | | | | | | | | | | | |
|---|---|---|---|---|---|---|---|---|---|---|---|---|---|---|---|---|---|
| *: Value obtained when non-addition of RF (0 IU/mL) is set at 100% | | | | | | | | | | | | | | | | | |

**[Table 11]**

| Storage conditions | | Filling in whole vessel/leaving at rest | | | | | | | | [Table 11] Filling in half of vessel/shaking | | | | | | | |
|---|---|---|---|---|---|---|---|---|---|---|---|---|---|---|---|---|---|
| pH | | 5.6 | 6.0 | 6.2 | 6.4 | 6.7 | 6.9 | 7.2 | 7.4 | 5.6 | 6.0 | 6.2 | 6.4 | 6.7 | 6.9 | 7.2 | 7.4 |
| KL-6(U/mL) | | | | | | | | | | | | | | | | | |
| Hb mg/dL | 0 | 387 | 417 | 416 | 417 | 434 | 438 | 442 | 446 | 373 | 412 | 425 | 422 | 434 | 442 | 450 | 467 |
| | 100 | 388 | 414 | 411 | 417 | 424 | 437 | 450 | 450 | 390 | 412 | 423 | 425 | 436 | 434 | 444 | 468 |
| | 200 | 393 | 416 | 427 | 417 | 427 | 439 | 447 | 442 | 380 | 403 | 417 | 418 | 431 | 435 | 442 | 458 |
| | 300 | 395 | 404 | 418 | 417 | 434 | 436 | 443 | 442 | 383 | 411 | 422 | 411 | 433 | 429 | 447 | 466 |
| | 400 | 399 | 418 | 412 | 427 | 429 | 437 | 439 | 440 | 380 | 401 | 408 | 418 | 426 | 431 | 436 | 460 |
| | 500 | 381 | 405 | 412 | 416 | 429 | 424 | 434 | 440 | 386 | 402 | 412 | 412 | 428 | 433 | 433 | 462 |

| Recovery per centage (%)* | | | | | | | | | | | | | | | | | |
|---|---|---|---|---|---|---|---|---|---|---|---|---|---|---|---|---|---|
| Hb mg/dL | 0 | 100% | 100% | 100% | 100% | 100% | 100% | 100% | 100% | 100% | 100% | 100% | 100% | 100% | 100% | 100% | 100% |
| | 100 | 100% | 99% | 99% | 100% | 98% | 100% | 102% | 101% | 105% | 100% | 100% | 101% | 101% | 98% | 99% | 100% |
| | 200 | 102% | 100% | 103% | 100% | 98% | 100% | 101% | 99% | 102% | 98% | 98% | 99% | 99% | 98% | 98% | 98% |
| | 300 | 102% | 97% | 100% | 100% | 100% | 100% | 100% | 99% | 103% | 100% | 99% | 97% | 100% | 97% | 99% | 100% |
| | 400 | 103% | 100% | 99% | 102% | 99% | 100% | 99% | 99% | 102% | 97% | 96% | 99% | 98% | 97% | 97% | 99% |
| | 500 | 98% | 97% | 99% | 100% | 99% | 97% | 98% | 99% | 103% | 98% | 97% | 98% | 99% | 98% | 96% | 99% |

| | | | | | | | | | | | | | | | | | |
|---|---|---|---|---|---|---|---|---|---|---|---|---|---|---|---|---|---|
| *: Value obtained when non-addition of Hb (0 mg/dL) is set at 100% | | | | | | | | | | | | | | | | | |

As is clear from Table 10, it was found that the interference reaction of a rheumatoid factor was suppressed in the pH range from pH 5.6 to 7.4, but the suppressive effect was decreased at pH 7.2 or more. However, in the pH range from pH 5.6 to 7.4, a decrease in the effect of suppressing the interference reaction of the rheumatoid factor was not observed even under preservation conditions for promoting the oxidation of a sulfite and ascorbate.

Moreover, as is clear from Table 11, the influence of pH on the influence of hemoglobin was not observed, and a difference caused by preservation conditions was not found, either. The pH of an immunoassay reagent prepared by mixing ascorbate into a sulfite is preferably pH 7.4 or less, more preferably pH 5.6 to 7.4, and further preferably pH 5.6 to 6.9.

### Example 9 Influence of concentration of thiol compound

The concentration of sodium sulfite was set at 250 mM, and the pH was set at pH 6.7. Then, the influence of the concentration of 2-mercaptoethylamine on the interference reaction of a rheumatoid factor and the influence of hemoglobin was evaluated.

### (1) Reagents

As a first reagent, a 50 mM PIPES buffer (pH 6.7) containing 250 mM sodium sulfite and 2-mercaptoethylamine (0 (comparative example), 10, 50, or 100 mM) was prepared. Moreover, reagents containing 10 mM or 50 mM 2-mercaptoethylamine, from which sodium sulfite was removed, were also prepared. Regarding a second reagent, the same second reagent as that used in Example 1 was used.

### (2) Measurement specimens

Measurement specimens were prepared in the same manner as that of Example 8.

### (3) Evaluation method

The specimens were measured in the same manner as that of Example 1. The results are shown in Table 12 and Table 13.

**[Table 12]**

| Concentration of Na sulfite | | 250mM | | | | Not added | |
|---|---|---|---|---|---|---|---|
| Concentration of 2-MEA | | Comp. Ex. | 10mM | 50mM | 100mM | 10mM | 50mM |
| Measurement value of KL-6 (U/mL) | | | | | | | |
| RF | 0 | 1243 | 1236 | 1166 | 1170 | 1237 | 1150 |
| IU/mL | 100 | 1206 | 1197 | 1170 | 1180 | | 1156 |
| | 200 | 1210 | 1196 | 1174 | 1183 | 1318 | 1156 |
| | 300 | 1215 | 1202 | 1169 | 1181 | | 1166 |
| | 400 | 1209 | 1201 | 1172 | 1186 | | 1167 |
| | 500 | 1224 | 1208 | 1179 | 1190 | 1399 | 1165 |
| Recovery percentage (%)* | | | | | | | |
| RF | 0 | 100% | 100% | 100% | 100% | 100% | 100% |
| IU/mL | 100 | 97% | 97% | 100% | 101% | | 101% |
| | 200 | 97% | 97% | 101% | 101% | 107% | 101% |
| | 300 | 98% | 97% | 100% | 101% | | 101% |
| | 400 | 97% | 97% | 101% | 101% | | 101% |
| | 500 | 99% | 98% | 101% | 102% | 113% | 101% |

| | | | | | | | |
|---|---|---|---|---|---|---|---|
| *: Value obtained when non-addition of RF (0 IU/mL) is set at 100% | | | | | | | |

**[Table 13]**

| Concentration of Na sulfite | | 250mM | | | | Not added | |
|---|---|---|---|---|---|---|---|
| Concentration of 2-MEA | | Comp. Ex. | 10mM | 50mM | 100mM | 10mM | 50mM |
| Measurement value of KL-6 (U/mL) | | | | | | | |
| Hb | 0 | 443 | 451 | 459 | 454 | 443 | 464 |
| mg/dL | 100 | 457 | 453 | 455 | 456 | | 466 |
| | 200 | 465 | 449 | 455 | 464 | 450 | 470 |
| | 300 | 476 | 451 | 455 | 458 | | 474 |
| | 400 | 485 | 448 | 454 | 461 | | 478 |
| | 500 | 496 | 446 | 453 | 460 | 453 | 474 |
| Recovery percentage (%)* | | | | | | | |
| Hb | 0 | 100% | 100% | 100% | 100% | 100% | 100% |
| mg/dL | 100 | 103% | 100% | 99% | 100% | | 100% |
| | 200 | 105% | 100% | 99% | 102% | 102% | 101% |
| | 300 | 107% | 100% | 99% | 101% | | 102% |
| | 400 | 109% | 99% | 99% | 102% | | 103% |
| | 500 | 112% | 99% | 99% | 101% | 102% | 102% |

| | | | | | | | |
|---|---|---|---|---|---|---|---|
| *: Value obtained when non-addition of Hb (0 mg/dL) is set at 100% | | | | | | | |

As is clear from Table 12, when 2-mercaptoethylamine was mixed into a sulfite, the influence of the concentration of 2-mercaptoethylamine on suppression of the interference reaction of a rheumatoid factor was not observed. In contrast, in the case of a reagent to which a sulfite was not added but 2-mercaptoethylamine alone was added, a difference was observed depending on the concentration of 2-mercaptoethylamine, and the interference reaction of the rheumatoid factor was found when 2-mercaptoethylamine was used at 10 mM.

As is clear from Table 13, in the comparative example not containing 2-mercaptoethylamine, the influence of hemoglobin was observed, whereas in the case of mixing 2-mercaptoethylamine into a sulfite, the influence of hemoglobin was not found, and the influence of the concentration of 2-mercaptoethylamine was not found, either.

### Example 10 Concentration of ascorbic acid

The concentration of sodium sulfite was set at 250 mM, and the pH was set at pH 6.7. Then, the influence of the concentration of ascorbic acid on the influence of hemoglobin was evaluated.

### (1) Reagents

As a first reagent, a 50 mM PIPES buffer (pH 6.7) containing 250 mM sodium sulfite and 1, 2, 5, 10, 30, 50, 100 or 300 mM sodium ascorbate was prepared. Regarding a second reagent, the same second reagent as that used in Example 1 was used.

### (2) Measurement specimens

Measurement specimens were prepared in the same manner as that for the Hb specimens of Example 8.

### (3) Evaluation method

The specimens were measured in the same manner as that of Example 1, and the influence of the concentration of sodium ascorbate on the influence of hemoglobin (Hb) was evaluated. The results are shown in Table 14.

**[Table 14]**

| | | Sodium ascorbate | | | | | | | | |
|---|---|---|---|---|---|---|---|---|---|---|
| | | Not added | 1mM | 2mM | 5mM | 10mM | 30mM | 50mM | 100mM | 300mM |
| Measurement value of KL-6 (U/mL) | | | | | | | | | | |
| Hb | 0 | 418 | 413 | 414 | 413 | 417 | 426 | 417 | 417 | 411 |
| mg/mL | 100 | 431 | 417 | 415 | 420 | 420 | 423 | 422 | 416 | 406 |
| | 200 | 428 | 416 | 419 | 416 | 418 | 427 | 422 | 414 | 416 |
| | 300 | 434 | 414 | 416 | 410 | 416 | 423 | 414 | 416 | 412 |
| | 400 | 439 | 419 | 411 | 415 | 420 | 425 | 418 | 416 | 421 |
| | 500 | 445 | 417 | 415 | 413 | 419 | 425 | 416 | 412 | 416 |
| Recovery percentage (%)* | | | | | | | | | | |
| Hb | 0 | 100% | 100% | 100% | 100% | 100% | 100% | 100% | 100% | 100% |
| mg/mL | 100 | 103% | 101% | 100% | 102% | 101% | 99% | 101% | 100% | 99% |
| | 200 | 102% | 101% | 101% | 101% | 100% | 100% | 101% | 99% | 101% |
| | 300 | 104% | 100% | 100% | 99% | 100% | 99% | 99% | 100% | 100% |
| | 400 | 105% | 101% | 99% | 100% | 101% | 100% | 100% | 100% | 102% |
| | 500 | 106% | 101% | 100% | 100% | 100% | 100% | 100% | 99% | 101% |

| | | | | | | | | | | |
|---|---|---|---|---|---|---|---|---|---|---|
| *: Value obtained when non-addition of hemolyzed hemoglobin (0 mg/mL) is set at 100% | | | | | | | | | | |

As is clear from Table 14, it was found that the influence of hemoglobin observed in non-addition of ascorbic acid could be completely suppressed by addition of sodium ascorbate at a concentration of 1 mM or more. In addition, it was also found that, even in a case where sodium ascorbate was added at a concentration of 300 mM, a precise measurement could be performed.

### Example 11 Confirmation of lower limit of additive concentration of reducing substance

The concentration of sodium sulfite was set at 250 mM, and the pH was set at pH6.7. Then, the lower limit of the additive concentration of sodium ascorbate or 2-mercaptoethylamine (2-MEA) used as a reducing substance was evaluated based on the effects thereof on changes over time in the absorption spectrum of hemoglobin.

### (Reagents)

First reagents were prepared in the same manner as that of Example 5, with the exception that the concentration of sodium ascorbate was set at 0.01 mM or 0.1 mM and the concentration of 2-mercaptoethylamine was set at 0.1 mM or 1.0 mM. Regarding a second reagent, the same second reagent as that used in Example 4 was used.

### (2) Measurement specimens

Measurement specimens were prepared in the same manner as that of Example 4.

### (3) Evaluation method

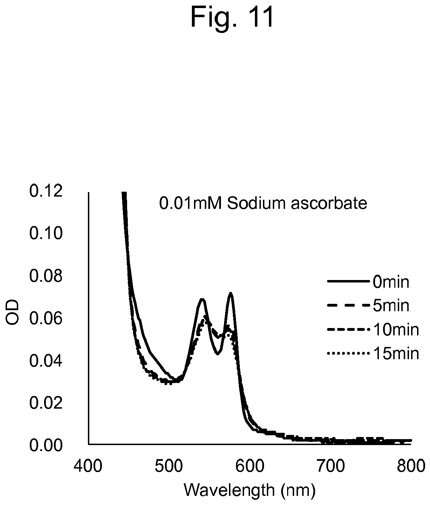

Changes over time in the absorption spectrum of hemoglobin were measured in the same manner as that of Example 4. The results are shown in Figure 11 to Figure 14.

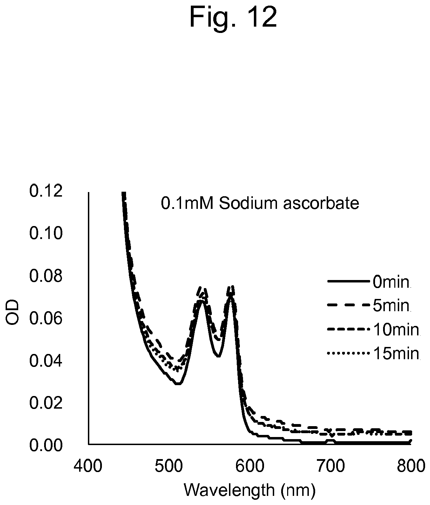

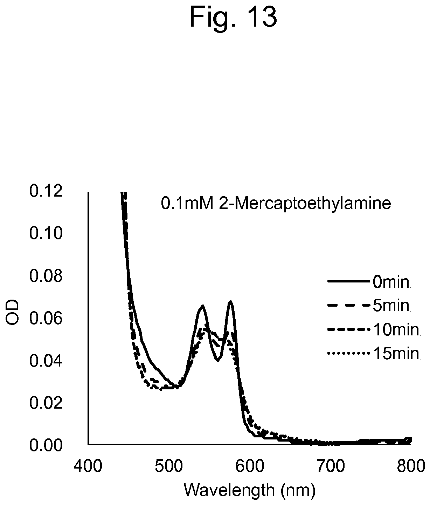

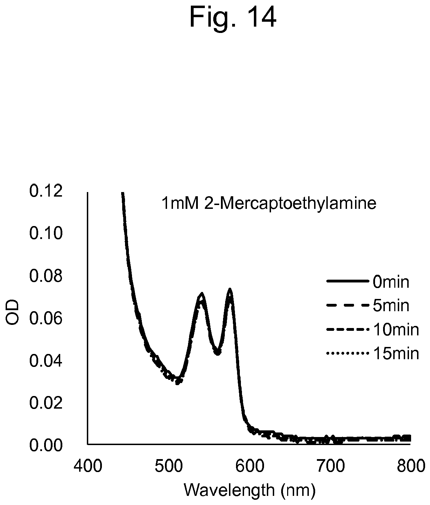

As shown in Figure 13 and Figure 14, it was found that changes over time in the absorption spectrum of hemoglobin were suppressed by addition of 2-mercaptoethylamine at 1 mM. Moreover, as shown in Figure 12, changes over time in the absorption spectrum of hemoglobin were somewhat observed, when 0.1 mM sodium ascorbate was added. However, such changes over time were small in comparison to addition of the same concentration of 2-mercaptoethylamine. Accordingly, it is considered that changes over time in the absorption spectrum of hemoglobin are suppressed by addition of 1 mM sodium ascorbate, as in the case of 2-mercaptoethylamine.

### Example 12 Evaluation of stability of reagent containing sulfite and ascorbate

A first reagent, to which sodium sulfite and ascorbic acid had been added, was stored at 4°C for 12 months. Thereafter, a human serum specimen containing an interference substance was measured, while the first reagent immediately after the preparation thereof was used as a control. Then, the stability of the effect of suppressing the interference reaction was evaluated.

### (1) Reagents

As a first reagent, a 50 mM PIPES buffer (pH 6.7) containing 250 mM sodium sulfite and 10 mM sodium ascorbate was prepared. In addition, another first reagent was prepared by removing sodium ascorbate from the composition of the aforementioned first reagent. Regarding a second reagent, the same second reagent as that used in Example 1 was used.

### (2) Measurement specimens

A human serum specimen containing a heterophile antibody (HARA, HAGA, HAMA or HAHA) and/or a rheumatoid factor was used as a specimen.

### (3) Evaluation method

The specimens were measured in the same manner as that of Example 1. The results are shown in Table 15.

**[Table 15]**

| | | Immediately after preparation | | | 12 months | | | Specimen information (heterophile antibody, etc.) | | | | |
|---|---|---|---|---|---|---|---|---|---|---|---|---|
| Na ascorbate | | Not added | Not added | 10mM | Not added | Not added | 10mM | HARA | HAGA | HAMA | HAHA | RF IU/mL |
| pH | | 7.4 | 6.7 | 6.7 | 7.4 | 6.7 | 6.7 | | | | | |
| Measurement value of KL-6 (U/mL) | | | | | | | | | | | | |
| Serum | 1 | 841 | 805 | 782 | 866 | 811 | 804 | | + | | | 75 |
| | 2 | 663 | 618 | 606 | 702 | 611 | 630 | + | ++ | ++ | ++ | 611 |
| | 3 | 1243 | 1189 | 1185 | 1289 | 1241 | 1237 | | | | | 100 |
| | 4 | 350 | 346 | 354 | 353 | 347 | 344 | | + | + | | 325 |
| | 5 | 436 | 426 | 437 | 437 | 432 | 426 | | | ++ | | 177 |
| | 6 | 675 | 641 | 635 | 690 | 641 | 636 | ++ | +++ | ++ | ++ | 1495 |
| | 7 | 459 | 449 | 458 | 469 | 458 | 451 | +++ | | +++ | ++ | 1859 |
| | 8 | 643 | 631 | 643 | 647 | 637 | 634 | | | | | 56 |
| | 9 | 677 | 623 | 634 | 706 | 635 | 635 | ++ | +++ | + | ++ | 1764 |
| | 10 | 321 | 321 | 320 | 306 | 308 | 308 | | | | | 60 |
| | 11 | 914 | 895 | 901 | 907 | 896 | 885 | | | | | 13 |
| | 12 | 552 | 538 | 527 | 565 | 534 | 527 | ++ | | + | | 479 |
| | 13 | 865 | 857 | 832 | 868 | 853 | 850 | ++ | | ++ | ++ | 515 |
| | 14 | 728 | 673 | 666 | 740 | 675 | 676 | ++ | ++ | + | ++ | 1033 |
| | 15 | 530 | 518 | 507 | 522 | 517 | 506 | + | | | | 282 |
| | 16 | 934 | 897 | 898 | 931 | 916 | 915 | ++ | | ++ | + | 177 |
| | 17 | 348 | 346 | 339 | 356 | 348 | 343 | | + | | | 399 |
| | 18 | 789 | 767 | 766 | 789 | 792 | 785 | ++ | | ++ | + | 323 |

As shown in Table 15, the measurement value of the serum specimen containing a heterophile antibody and/or a rheumatoid factor, which was measured using a first reagent that had been stored at 4°C for 12 months, was the same level as the measurement value of the serum specimen, which was measured using a first reagent immediately after the preparation thereof. Thus, even after the first reagent was stored at 4°C for 12 months, a decrease in the effect of suppressing the interference reaction was not observed, and thus, the reagent was stable for a long period of time. Moreover, a difference in the stability depending on the presence or absence of sodium ascorbate was not observed, either. However, when the reagent does not contain ascorbic acid, the influence of hemoglobin is found, as described in Example 5 and the like.

### Example 13 Effects of measurement reagent on MMP-3

Furthermore, the measurement target substance was changed to MMP-3, and the effects of the present invention thereon were confirmed.

### (1) Reagents

As a first reagent, a 50 mM PIPES buffer (pH 6.7) containing 250 mM sodium sulfite and 10 mM sodium ascorbate was prepared. In addition, another first reagent was prepared by removing sodium ascorbate from the composition of the aforementioned first reagent. Moreover, a further first reagent, which is the aforementioned first reagent from which sodium ascorbate was removed, in which the PIPES buffer (pH 6.7) was replaced with the HEPES buffer (pH 7.4), was also prepared.

As a second reagent, a 50 mM HEPES buffer (pH 7.4) containing an anti-MMP-3 antibody-immobilizing polystyrene latex solution was prepared.

The above-described anti-MMP-3-immobilizing latex solution was prepared according to a known method. Specifically, the solution was prepared by mixing an anti-MMP-3 antibody with polystyrene latex so that the anti-MMP-3 antibody was adsorbed on the surface of the polystyrene latex.

### (2) Measurement specimens

A heterophile antibody- or rheumatoid factor-positive human serum specimen, and the same Hb specimens as those in Example 8 were prepared.

### (3) Evaluation method

To 2.4 µL of the specimen, 120 µL of the first reagent was mixed, and the obtained mixture was then incubated at 37°C for 5 minutes. Thereafter, 40 µL of the second reagent was mixed into the above-obtained mixed solution, and the thus obtained mixture was reacted at 37°C. Then, for approximately 5 minutes after the mixing of the second reagent, changes in the absorbance were measured at two wavelengths, namely, at a main wavelength of 570 nm and at a complementary wavelength of 800 nm. It is to be noted that a series of measurements were carried out using Hitachi 7180 automatic analyzer (Hitachi High-Technologies Corporation).

In order to quantify the concentration of MMP-3, the concentration of MMP-3 in the specimen was calculated from a calibration curve obtained by measuring a standard substance of MMP-3. The results are shown in Table 16 and Table 17.

**[Table 16]**

| Na ascorbate | | Not added | Not added | 10mM | Remarks |
|---|---|---|---|---|---|
| pH | | 7.4 | 6.7 | 6.7 | |
| Measurement value of MMP-3 (ng/mL) | | | | | |
| Serum | 1 | 90 | 30 | 29 | High RF value |
| | 2 | 99 | 54 | 55 | HAMA |
| | 3 | 170 | 168 | 164 | |
| | 4 | 56 | 51 | 53 | |
| | 5 | 69 | 67 | 66 | |
| | 6 | 77 | 76 | 75 | |
| | 7 | 68 | 66 | 67 | |
| | 8 | 440 | 434 | 431 | |
| | 9 | 544 | 553 | 549 | |
| | 10 | 574 | 550 | 564 | |
| | 11 | 597 | 596 | 600 | |
| | 12 | 81 | 53 | 55 | HAMA |
| | 13 | 564 | 98 | 91 | HAMA |
| | 14 | 51 | 54 | 52 | |
| | 15 | 56 | 53 | 55 | |
| | 16 | 29 | 28 | 25 | |
| | 17 | 208 | 209 | 205 | |
| | 18 | 136 | 130 | 131 | |
| | 19 | 35 | 33 | 31 | |
| | 20 | 46 | 44 | 47 | |
| | 21 | 41 | 43 | 42 | |
| | 22 | 393 | 396 | 391 | |
| | 23 | 29 | 32 | 29 | |

**[Table 17]**

| Na ascorbate | | Not added | Not added | 10mM |
|---|---|---|---|---|
| pH | | 7.4 | 6.7 | 6.7 |
| Measurement value of MMP-3 (ng/mL) | | | | |
| Hb | 0 | 84 | 84 | 84 |
| mg/dL | 100 | 85 | 83 | 83 |
| | 200 | 84 | 82 | 84 |
| | 300 | 82 | 81 | 83 |
| | 400 | 84 | 79 | 82 |
| | 500 | 83 | 74 | 82 |

| Recovery percentage (%)* | | | | |
|---|---|---|---|---|
| | 0 | 100% | 100% | 100% |
| | 100 | 100% | 99% | 99% |
| | 200 | 100% | 97% | 100% |
| | 300 | 97% | 96% | 99% |
| | 400 | 99% | 94% | 98% |
| | 500 | 99% | 87% | 98% |

| | | | | |
|---|---|---|---|---|
| *: Value obtained when non-addition of Hb (0 mg/dL) is set at 100% | | | | |

As is clear from Table 16, regardless of the presence or absence of sodium ascorbate, an interference reaction caused by an interference substance could be suppressed by setting the pH of the first reagent at pH 6.7. On the other hand, when the pH of the first reagent was set at pH 7.4, such an interference reaction caused by an interference substance could not be sufficiently suppressed in serums (serum 1, serum 2, serum 12, and serum 14) in which the interference substance was contained in a high concentration, and thus, the measurement value of MMP-3 increased.

As is clear from Table 17, even in the case of MMP-3, when the pH of the first reagent was set at pH 6.7 and ascorbate was not added, the influence of hemoglobin was observed. In contrast, the influence of hemoglobin could be suppressed by addition of ascorbate.

### Industrial Applicability

As described above, according to the immunoassay method and uses of the present invention, it becomes possible to provide an immunoassay method and the use of an immunoassay reagent capable of precisely measuring a measurement target, which can sufficiently suppress interference effects caused by an interference substance, such as a heterophile antibody or a rheumatoid factor, for a long period of time, even in a case where the measurement specimen contains the interference substance in a high concentration, and are not affected with hemoglobin.

## Claims

1. An immunoassay method of carrying out an antigen-antibody reaction under suppression of interference reaction of a rheumatoid factor using an immunoassay reagent in the presence of a sulfite, wherein the immunoassay reagent comprises a sulfite and a reducing substance.

2. The immunoassay method according to claim 1, wherein the concentration of the reducing substance is from 1 mM to 300 mM.

3. The immunoassay method according to claim 1 or claim 2, wherein the concentration of the sulfite is from 50 mM to 600 mM.

4. The immunoassay method according to any one of claim 1 to claim 3, wherein the pH of the immunoassay reagent comprising the sulfite and the reducing substance is from pH 5.6 to 7.4.

5. The immunoassay method according to any one of claim 1 to claim 4, wherein the reducing substance is any one or more reducing substances selected from ascorbic acid or a salt thereof and a thiol compound.

6. The immunoassay method according to claim 5, wherein the thiol compound is any one or more thiol compounds selected from 2-mercaptoethylamine, 2-dimethylaminoethanethiol, 2-mercaptoethanol, 3-mercaptopropanol, dithiothreitol, cysteine, N-acetylcysteine, reduced glutathione, and thiophenol.

7. The immunoassay method according to any one of claim 1 to claim 6, comprising a step of mixing a first reagent comprising the immunoassay reagent with a second reagent comprising an antibody or an antigen reacting with a measurement target, or a carrier particle immobilizing an antibody or an antigen reacting with a measurement target, wherein the measurement target is quantitatively or qualitatively measured.

8. The immunoassay method according to any one of claim 1 to claim 7, which is latex agglutination immunoassay.

9. Use of an immunoassay reagent in an immunoassay method of carrying out an antigen-antibody reaction under suppression of interference reaction of a rheumatoid factor in the presence of a sulfite, wherein the immunoassay reagent comprises a sulfite and a reducing substance.

10. Use according to claim 9, wherein the concentration of the reducing substance is from 1 mM to 300 mM.

11. Use according to claim 9 or claim 10, wherein the concentration of the sulfite is from 50 mM to 600 mM.

12. Use according to any one of claim 9 to claim 11, wherein the pH of the immunoassay reagent comprising the sulfite and the reducing substance is from pH 5.6 to 7.4.

13. Use according to any one of claim 9 to claim 12, wherein the reducing substance is any one or more reducing substances selected from ascorbic acid or a salt thereof and a thiol compound.

14. Use according to claim 13, wherein the thiol compound is any one or more thiol compounds selected from 2-mercaptoethylamine, 2-dimethylaminoethanethiol, 2-mercaptoethanol, 3-mercaptopropanol, dithiothreitol, cysteine, N-acetylcysteine, reduced glutathione, and thiophenol.

15. Use according to any one of claim 9 to claim 14, wherein the immunoassay method is latex agglutination immunoassay.

16. Use of an immunoassay reagent kit in an immunoassay method of carrying out an antigen-antibody reaction under suppression of interference reaction of a rheumatoid factor in the presence of a sulfite, comprising a first reagent comprising the immunoassay reagent as defined in any one of claim 9 to claim 15, and a second reagent comprising an antibody or an antigen reacting with a measurement target, or a carrier particle immobilizing an antibody or an antigen reacting with a measurement target.

## Patentansprüche

1. Immunoassay-Verfahren zum Durchführen einer Antigen-Antikörper-Reaktion unter Unterdrückung einer Interferenzreaktion eines Rheumafaktors unter Verwendung eines Immunoassay-Reagens in Gegenwart eines Sulfits, wobei das Immunoassay-Reagens ein Sulfit und eine reduzierende Substanz umfasst.

2. Immunoassay-Verfahren nach Anspruch 1, wobei die Konzentration der reduzierenden Substanz 1 mM bis 300 mM beträgt.

3. Immunoassay-Verfahren nach Anspruch 1 oder 2, wobei die Konzentration des Sulfits 50 mM bis 600 mM beträgt.

4. Immunoassay-Verfahren nach einem der Ansprüche 1 bis 3, wobei der pH-Wert des Immunoassay-Reagens, das das Sulfit und die reduzierende Substanz umfasst, 5,6 bis 7,4 beträgt.

5. Immunoassay-Verfahren nach einem der Ansprüche 1 bis 4, wobei die reduzierende Substanz eine oder mehrere reduzierende Substanzen ist, ausgewählt aus Ascorbinsäure oder einem Salz davon und einer Thiolverbindung.

6. Immunoassay-Verfahren nach Anspruch 5, wobei die Thiolverbindung eine oder mehrere Thiolverbindungen ist, ausgewählt aus 2-Mercaptoethylamin, 2-Dimethylaminoethanthiol, 2-Mercaptoethanol, 3-Mercaptopropanol, Dithiothreitol, Cystein, N-Acetylcystein, reduziertem Glutathion und Thiophenol.

7. Immunoassay-Verfahren nach einem der Ansprüche 1 bis 6, umfassend einen Schritt des Mischen eines ersten Reagens, umfassend das Immunoassay-Reagens, mit einem zweiten Reagens, umfassend einen Antikörper oder ein Antigen, das mit einem Messziel reagiert, oder ein Trägerpartikel, das einen Antikörper oder ein Antigen immobilisiert, das mit einem Messziel reagiert, wobei das Messziel quantitativ oder qualitativ gemessen wird.

8. Immunoassay-Verfahren nach einem der Ansprüche 1 bis 7, welches Latex-Agglutinations-Immunoassay ist.

9. Verwendung eines Immunoassay-Reagens in einem Immunoassay-Verfahren zum Durchführen einer Antigen-Antikörper-Reaktion unter Unterdrückung einer Interferenzreaktion eines Rheumafaktors in Gegenwart eines Sulfits, wobei das Immunoassay-Reagens ein Sulfit und eine reduzierende Substanz umfasst.

10. Verwendung nach Anspruch 9, wobei die Konzentration der reduzierenden Substanz 1 mM bis 300 mM beträgt.

11. Verwendung nach Anspruch 9 oder 10, wobei die Konzentration des Sulfits 50 mM bis 600 mM beträgt.

12. Verwendung nach einem der Ansprüche 9 bis 11, wobei der pH-Wert des Immunoassay-Reagens, das das Sulfit und die reduzierende Substanz umfasst, 5,6 bis 7,4 beträgt.

13. Verwendung nach einem der Ansprüche 9 bis 12, wobei die reduzierende Substanz eine oder mehrere reduzierende Substanzen ist, ausgewählt aus Ascorbinsäure oder einem Salz davon und einer Thiolverbindung.

14. Verwendung nach Anspruch 13, wobei die Thiolverbindung eine oder mehrere Thiolverbindungen ist, ausgewählt aus 2-Mercaptoethylamin, 2-Dimethylaminoethanthiol, 2-Mercaptoethanol, 3-Mercaptopropanol, Dithiothreitol, Cystein, N-Acetylcystein, reduziertem Glutathion und Thiophenol.

15. Verwendung nach einem der Ansprüche 9 bis 14, wobei das Immunoassay-Verfahren ein Latex-Agglutinations-Immunoassay ist.

16. Verwendung eines Immunoassay-Reagens-Kits in einem Immunoassay-Verfahren zum Durchführen einer Antigen-Antikörper-Reaktion unter Unterdrückung einer Interferenzreaktion eines Rheumafaktors in Gegenwart eines Sulfits, umfassend ein erstes Reagens, umfassend das Immunoassay-Reagens, wie in einem der Ansprüche 9 bis 15 definiert, und ein zweites Reagens, umfassend einen Antikörper oder ein Antigen, das mit einem Messziel reagiert, oder ein Trägerpartikel, das einen Antikörper oder ein Antigen immobilisiert, das mit einem Messziel reagiert.

## Revendications

1. Procédé de dosage immunologique de mise en œuvre d'une réaction antigène-anticorps avec suppression d'une réaction d'interférence d'un facteur rhumatoïde utilisant un réactif de dosage immunologique en la présence d'un sulfite, le réactif de dosage immunologique comprenant un sulfite et une substance réductrice.

2. Procédé de dosage immunologique selon la revendication 1, la concentration de la substance réductrice étant de 1 mM à 300 mM.

3. Procédé de dosage immunologique selon la revendication 1 ou la revendication 2, la concentration du sulfite étant de 50 mM à 600 mM.

4. Procédé de dosage immunologique selon l'une quelconque parmi la revendication 1 à la revendication 3, le pH du réactif de dosage immunologique comprenant le sulfite et la substance réductrice étant de pH 5,6 à 7,4.

5. Procédé de dosage immunologique selon l'une quelconque parmi la revendication 1 à la revendication 4, la substance réductrice étant l'une quelconque ou plusieurs substances réductrices choisies parmi l'acide ascorbique ou un sel correspondant et un composé de type thiol.

6. Procédé de dosage immunologique selon la revendication 5, le composé de type thiol étant l'un quelconque ou plusieurs composés de type thiol choisis parmi la 2-mercaptoéthylamine, le 2-diméthylaminoéthanethiol, le 2-mercaptoéthanol, le 3-mercaptopropanol, le dithiothréitol, la cystéine, la N-acétylcystéine, le glutathion réduit, et le thiophénol.

7. Procédé de dosage immunologique selon l'une quelconque parmi la revendication 1 à la revendication 6, comprenant une étape de mélange d'un premier réactif comprenant le réactif de dosage immunologique avec un deuxième réactif comprenant un anticorps ou un antigène réagissant avec une cible de mesure, ou une particule support immobilisant un anticorps ou un antigène réagissant avec une cible de mesure, la cible de mesure étant mesurée quantitativement ou qualitativement.

8. Procédé de dosage immunologique selon l'une quelconque parmi la revendication 1 à la revendication 7, qui est un dosage immunologique d'agglutination de latex.

9. Utilisation d'un réactif de dosage immunologique dans un procédé de dosage immunologique de mise en œuvre d'une réaction antigène-anticorps avec suppression d'une réaction d'interférence d'un facteur rhumatoïde en la présence d'un sulfite, le réactif de dosage immunologique comprenant un sulfite et une substance réductrice.

10. Utilisation selon la revendication 9, la concentration de la substance réductrice étant de 1 mM à 300 mM.

11. Utilisation selon la revendication 9 ou la revendication 10, la concentration du sulfite étant de 50 mM à 600 mM.

12. Procédé de dosage immunologique selon l'une quelconque parmi la revendication 9 à la revendication 11, le pH du réactif de dosage immunologique comprenant le sulfite et la substance réductrice étant de pH 5,6 à 7,4.

13. Procédé de dosage immunologique selon l'une quelconque parmi la revendication 9 à la revendication 12, la substance réductrice étant l'une quelconque ou plusieurs substances réductrices choisies parmi l'acide ascorbique ou un sel correspondant et un composé de type thiol.

14. Utilisation selon la revendication 13, le composé de type thiol étant l'un quelconque ou plusieurs composés de type thiol choisis parmi la 2-mercaptoéthylamine, le 2-diméthylaminoéthanethiol, le 2-mercaptoéthanol, le 3-mercaptopropanol, le dithiothréitol, la cystéine, la N-acétylcystéine, le glutathion réduit, et le thiophénol.

15. Utilisation selon l'une quelconque parmi la revendication 9 à la revendication 14, le procédé de dosage immunologique étant un dosage immunologique d'agglutination de latex.

16. Utilisation d'un kit de réactifs de dosage immunologique dans un procédé de dosage immunologique de mise en œuvre d'une réaction antigène-anticorps avec suppression d'une réaction d'interférence d'un facteur rhumatoïde en la présence d'un sulfite, comprenant un premier réactif comprenant le réactif de dosage immunologique tel que défini dans l'une quelconque parmi la revendication 9 à la revendication 15, et un deuxième réactif comprenant un anticorps ou un antigène réagissant avec une cible de mesure, ou une particule support immobilisant un anticorps ou un antigène réagissant avec une cible de mesure.
